# EUROPEAN PATENT APPLICATION

(11) **EP 2 995 607 A1**
(43) Date of publication of application: **16.03.2016**
(21) Application number: 14184259.1
(22) Date of filing: 10.09.2014
(51) Int. Cl.: C07C 209/14, C07C 209/16

(54) **Direct amination of alcohols with heteropoly acids**

(71) Applicant: Taminco bvba, 9000 Gent (BE)
(72) Inventor: Moonen, Kristof, 9000 Gent (BE); Ulrichts, Dieter, 9000 Gent (BE); Roose, Peter, 9831 Deurle (BE); Paul, Sébastien, 59158 Thun-Saint-Amand (FR); Katryniok, Benjamin, 62410 Meurchin (FR); Safariamin, Maryam, 92350 Le Plessis Robinson (FR); Dumeignil, Franck, 59273 Fretin (FR)
(74) Representative: Brantsandpatents bvba

(57) **Abstract**

The present invention describes a process for preparing amines by reacting a alcohol with an aminating agent in presence of a catalyst, whereby said catalyst is a heteropoly acid comprising a transition metal, and/or a salt thereof. Preference is given to using glycerol. The preferred transition metal of said heteropoly acid is preferably selected from the group of tungsten, molybdenum and/or vanadium. Said heteropoly acid further comprises a non-metal, preferably from the group of silicon, phosphorus, germanium and/or vanadium. The heteropoly acid is preferably supported by silica. This invention provides industrial important aminoderivatives.

## Description

### TECHNICAL FIELD

The present invention relates to a process for preparing amines by reacting an alcohol with an aminating agent in presence of a catalyst, whereby said catalyst is a heteropoly acid and/or a salt thereof.

More specifically, the present invention relates to a process for the direct amination reaction of glycerol whereby said heteropoly compound is comprised of a cesium salt.

### INTRODUCTION

Amines are important products in organic synthesis. They are used in numerous applications such as pharmaceuticals, crop protection (pesticides and insecticides), surfactants and detergents, food processing, solvents, fuel additives, or for oil & gas treatment.

Amines are generally employed as building blocks for the production of surfactants, medicaments and crop protection compositions, stabilizers, polymers, hardeners for epoxy resins, catalysts for polyurethanes, intermediates for preparing quaternary ammonium compounds, plasticizers, corrosion inhibitors, synthetic resins, ion exchangers, textile assistants, dyes, solvents, neutralizing agents, biocides, vulcanization accelerators and/or emulsifiers.

Processes for the preparation of amines from alcohols are known from literature. US 2010/0240894 describes a process for preparing amines by reacting glycerol with hydrogen and an aminating agent from the group of ammonia and primary and secondary amines in the presence of a catalyst at a temperature from 100°C to 400°C and at a pressure from 0.01 to 40 MPa (from 0.1 to 400 bar). Preference is given to the use of glycerol derived from renewable raw materials. The catalyst preferably comprises one metal or a plurality of metals or one or more oxygenated compounds of the metals of groups 8 and/or 9 and/or 10 and/or 11 of the Periodic Table of the Elements. It further discloses the use of the reaction products as an additive in cement or concrete production. The process disclosed is applied to synthesis of the compounds 1,2,3-triaminopropane, 2-aminomethyl-6-methylpiperazine, 2,5-bis(aminomethyl)piperazine and 2,6-bis(aminomethyl)-piperazine.

Fisher et al. (Catalyst Today 1997, 37, 167-189) reviewed the heterogeneous catalytic transformation of bi- and polyfunctional alcohols to the corresponding linear and branched acyclic amines. The aminating components are mainly ammonia, but also primary and secondary amines. Typical catalysts can be divided into two main groups: supported metal and multimetallic hydrogenation catalysts and solid acids.

Many side reactions can occur in the amination reaction of diols and triols, as compared to that of monofunctional alcohols. The development of an economic process for the synthesis of aminoalcohols, diamines and triamines is demanding.

Hernandez et al. (Applied Catalysis A: General 467, 2013, 315-324) describes the catalytic performance of lanthanum based catalysts: LaNiO₃ and La₂O₃ were evaluated in the gas phase transformation of glycerol. The reaction was investigated in the temperature range of 300 to 700°C. At 400°C and 500°C, the production of compounds in liquid phase is favoured while at high temperature gases such as carbon monoxide, carbon dioxide and hydrogen are mainly formed. Catalysts used in this study favour the production of hydroxyacetone.

EP 2 595 746 describes a heteropoly acid compound catalyst composition, a method of making the catalyst composition and a process for the oxidation of saturated and/or unsaturated aldehydes to unsaturated carboxylic acids using the catalyst composition. The catalyst composition is a heteropoly acid compound containing molybdenum, vanadium, phosphorus, cesium, bismuth, copper, and antimony.

Although there is a strong industrial interest in acyclic di-, tri- and polyamines, little successful reports have been made on processes for transforming diols, triols and polyols to the corresponding amines. Diamines and polyamines, especially ethylene diamine and polyethylene diamine homologues, find a broad range of commercial applications. One of the problems with the reported amination reactions is the lack of conversion, selectivity and/or yield of the desired amine product, which are far below the economically acceptable level. Other problems relate to the stability and selectivity of the catalyst, lifetime and recyclability of said catalyst during processing, and difficult reaction conditions to obtain reasonable conversion of starting products to the desired amine compounds.

### SUMMARY OF THE INVENTION

The present invention provides a solution to at least one of the above mentioned problems. The invention wants to avoid harsh and costly process conditions occurring in methods known thus far.

The present invention provides a process for preparing amines by reacting an alcohol with an aminating agent in presence of a catalyst, whereby said catalyst is a heteropoly acid comprising one or more central atoms (X) and addenda atoms (M, N), whereby said addenda atoms comprise one or more transition metals, and/or a salt of said heteropoly acid. Surprisingly, processes according to the invention exhibit a reduced formation of cokes on said employed heteropoly acids, better reaction yields and selectivity, and longevity of the catalyst.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. All percentages are to be understood as percentage by weight and are abbreviated as "%wt." or "% by weight", unless otherwise defined or unless a different meaning is obvious to the person skilled in the art from its use and in the context wherein it is used.

In a first aspect, the invention provides a process for preparing amines by reacting an alcohol with an aminating agent in presence of a catalyst, whereby said catalyst is a heteropoly acid comprising one or more central atoms (X) and addenda atoms (M, N), whereby said addenda atoms comprise one or more transition metals, and/or a salt of said heteropoly acid.

This is of interest because said process provides an advantageous route to amines, which are important products in organic synthesis and have a high commercial value. They are used in numerous applications such as pharmaceuticals, crop protection (pesticides and insecticides), food processing, solvents, surfactants and detergents, fuel additives, or for oil & gas treatment. A direct access route to amines is of economic importance.

One of the advantages of the above process, is that less cokes formation was observed during the process. In addition, the process yielded higher conversions and higher selectivity towards the desired product. Consequently, higher yields were obtained. Furthermore, an enhanced lifetime for the catalysts was observed, compared to classically used catalysts. Another advantage is that the catalysts can be easily recycled after removing the formed cokes by heating and oxidation of said cokes.

Due to formation of various reaction products, obtaining a high conversion, selectivity and yield of the desired amine end-product is a difficult object to achieve. It is desired to obtain sufficiently high selectivity to the desired product. Already with monofunctional alcohols side reactions can occur. Difficulties are amplified when using a di- or polyfunctional alcohol reactant and in many cases the selectivity to the desired product is far below the economically acceptable level.

The term "alcohol" as used within the scope of the invention refers to an organic compound comprising at least one hydroxyl group. More specifically, when used in a process according to the invention, the term "alcohol" refers to an organic compound comprising at least one hydroxyl group and at least one second functional organic group selected from the group of alcohols and amines. In a preferred embodiment, said alcohol is comprised of two or more carbon atoms, whereby said hydroxyl group and said functional organic group are located on distinct carbon atoms, more preferably on vicinal carbon atoms. Selected examples are glycol and monoethanolamine. More preferably, said alcohol comprises a polyol having two or more hydroxyl groups, which are preferably located in vicinal position relative to each other. Amination of polyols has attracted specific attention, due to the demand for diamines and polyamines, especially ethylene diamine and its polymers. In a more preferred embodiment, said alcohol is selected from the group comprising glycerol, ethylene glycol, propylene glycol, monoethanolamine, 1,4-butanediol and glycol ethers such as diethylene glycol.

The term "aminating agent" refers to an organic compound selected from the group consisting of ammonia, primary amines and secondary amines. As well as ammonia, it is equally possible to use primary or secondary amines as aminating agents. For example, the following mono- and dialkylamines may be used as aminating agents: methylamine, dimethylamine, ethylamine, diethylamine, n-propylamine, di-n-propylamine, isopropylamine, diisopropylamine, isopropylethylamine, n-butylamine, di-n-butylamine, s-butylamine, di-s-butylamine, isobutylamine, n-pentylamine, s-pentylamine, isopentylamine, n-hexylamine, s-hexylamine, isohexylamine, cyclohexylamine, aniline, toluidine, piperidine, morpholine and pyrrolidine. However, preference is given to using inexpensive aminating agents available on the industrial scale, such as ammonia, dimethylamine, methylamine or diethylamine. In a more preferred embodiment, said dialkylamine is dimethylamine. This is of interest because dimethylamine is inexpensive and available on the industrial scale.

Said process according to the first aspect of the invention is referred to by the term "direct amination" and is understood mechanistically, although the invention cannot be understood to be limited by the following explanation, to be a process that proceeds *via* a dehydration of the alcohol to an activated intermediate, before subsequent amination can take place on the same catalyst surface.

The term 'heteropoly acid' refers to a compound comprising a metal, a non-metal, an acidic proton and oxygen, which are more specifically configured within a structure comprised of an anionic part and one or more counter-cations. Said one or more counter-cations are preferably selected from the group comprising metal cations, protons and ammonium. Said anionic part is defined as a framework of central atoms (X) and addenda atoms (M, N) which are interconnected by oxygen bridges. Said central atoms (X) are in general selected from group 14 and/or 15 of the periodic table of elements, such as but not limited to silicon, phosphorus, germanium and arsenic. Said addenda atoms (M, N) are in general transition metals selected from the group 5, 6, 7, 8, 9, 10 and/or 11 of the periodic table of elements, such as but not limited to W, Mo, V, Ni, Co and Fe. Said anionic part can further be defined by one or more of the following structural formulas: [XM₁₂O₄₀]ⁿ⁻, also referred to as Keggin structure; [XM₆O₂₄]ⁿ⁻, also referred to as Anderson structure; and [X₂M₁₈O₆₂]ⁿ⁻, also referred to as Dawson structure. In a preferred embodiment, said heteropoly acid is comprised of an anionic part comprising a Keggin structure. The term "heteropoly acid" also refers to a compound as described above, whereby said structure comprises one or more MO₄ and/or MO₆ lacuna, also referred to as lacunary structures. Further definitions of the term "heteropoly acid" can be found in literature, such as in Chemical Reviews 1998, 98, 51-76, Yves P. Jeannin; in EP 1 439 908 and in WO 2008/153341, which definitions are hereby included by reference.

The inventors have found that heteropoly acids, especially those comprising a transition metal, and/or a salt thereof are especially suitable for mediating the direct amination of alcohols. This is of interest because heteropoly acids are environmentally friendly and economically viable Brönsted acids. Hereby, heteropoly acids offer the unique feature to enable fine modification of their acidity, and thereby of their catalytic properties, namely by varying the transition metal, the addenda atoms and the counter ions. The inventors have found that the use of heteropoly acids for the direct amination of alcohols has the advantage over other catalysts that there is less cokes formation on the catalyst. Eventually, higher yields and selectivity are obtained and said catalyst has a higher longevity during said process.

The term 'transition metal' refers to an element selected from the d-block of the Periodic Table of Elements. For clarity, the term 'transition metal' as used in a process according to the present invention does not refer to the f-block of the Periodic Table of Elements. Preferably, said transition metal is selected from group 3, 4, 5, 6 or 7.

In a preferred embodiment, the invention provides a process according to the first aspect of the invention, wherein said process is a gas phase process. This is advantageous since said processes performed in gas phase yields better conversions and selectivity to the desired products, as well as a reduced catalyst degradation. Said catalyst being essentially a solid compound, the reactions take place at the solid-gas interphase. In a more preferred embodiment, the invention provides a process according to the first aspect of the invention, wherein said gas phase process comprises a carrier gas, preferably an inert carrier gas.

According to certain embodiments, reacting the alcohol with the metal catalyst may occur under gas sparging. If a gas is used, any gas known to those skilled in the art may be acceptable for use herein. Examples of gasses that may be useful in certain embodiments of the present processes can include the noble gases (e.g. helium or argon), nitrogen, carbon dioxide, superheated steam, and combinations of any thereof. In certain embodiments, the gas may comprise nitrogen. Without being limited by theory, it is believed that the inclusion of a gas, in combination with the apt reaction temperature, can be beneficial because it can improve reaction yield and selectivity by enhancing mass transfer from and to the catalyst surface or reducing contact time between the catalyst and reagents.

In a preferred embodiment, said reaction is performed in absence of a reducing agent. As used herein, "reducing agent" refers to any element, compound, or combination of elements and/or compounds that reduces another species by either increasing the hydrogen content or decreasing the oxygen content of the other species.

Compounds to be understood as a reducing agent (such as a hydrogenation catalyst and hydrogen) may be any reducing agent known in the art. For example, such reduction reactions include hydrogenation with hydrogen gas and a hydrogenation catalyst; reduction with a hydride source, such as, but not limited to, sodium borohydride, acyloxyborohydrides, triacetoxy borohydride, cyanoborohydrides, and the like; dissolving metal reductions; and aluminium-mercury amalgam reductions. Specifically, the reducing agent may comprise hydrogen gas in the presence of a hydrogenation catalyst, such as a metal hydrogenation catalyst. This is of interest since in absence of hydrogen gas, it was found that good conversions and high selectivity were obtained for the non-reductive amination product. Furthermore, hydrogen gas is potentially explosive and can trigger undesired side reactions due to formation of metal hydrides. As an alternative to hydrogen gas, nitrogen gas can be employed as a carrier gas. Furthermore, no reducing or hydrogenative pretreatment of the catalyst is required before starting up the catalytic process.

In a preferred embodiment, the invention provides a process according to the first aspect of the invention, wherein said addenda atoms are selected from group 5, group 6 or group 7 of the periodic table. Examples of such elements or compounds thereof are, but not limited to, manganese, rhenium, chromium, molybdenum, tungsten, tantalum, niobium and vanadium. These metals are preferably comprised in the form of oxides, but can also be comprised as oxalates or pyrophosphates. In a more preferred embodiment, the invention provides a process according to the first aspect of the invention, wherein said addenda atoms (M, N) are selected from the group consisting of tungsten, molybdenum and vanadium, or any mixture thereof.

In a preferred embodiment, the invention provides a process according to the first aspect of the invention, wherein said central atoms (X) comprise one or more central atoms selected from group 14 or group 15 of the periodic table, or any mixture thereof. In a preferred embodiment, the invention provides a process according to the first aspect of the invention, wherein said central atoms (X) are selected from the group consisting of silicon, phosphorus, germanium and arsenic, or any mixture thereof.

In a preferred embodiment, the invention provides a process according to the first aspect of the invention, wherein said salt of said heteropoly acid comprises an element selected from group 1 and/or group 2, preferably rubidium and/or cesium.

This is advantageous since said salts of said heteropoly acids incorporate acidic sites of different nature and strength in the catalyst. Furthermore, said salts can provide the necessary electronic interaction between the heteropoly acid and any support material, which can be used to obtain increased conversion of alcohol, e.g. glycerol, compared to bulk catalysts. The inventors thus unexpectedly found that cesium-based heteropoly acids, as catalysts for the direct amination of alcohols with an aminating agent, show enhanced catalytic activity. In fact, whereas the strong and medium acid sites are occupied under the employed reaction temperature by chemisorbed aminating agent, weak sites are still accessible for the activation of the alcohol on the catalyst surface, which is supposed to be the first step of the direct amination mechanism.

In a preferred embodiment, the invention provides a process according to the first aspect of the invention, whereby said heteropoly acid is provided as a Keggin structure and/or a Dawson structure, most preferably a Keggin structure.

The heteropoly acid can include a Keggin or Dawson entity. In a more preferred embodiment, said heteropoly acid comprises a Keggin entity. Part of the molecular structure of heteropoly compounds containing a Keggin structure, is composed of one or more cells with a high centre of symmetry polymeric components. The structure self-assembles in acidic aqueous solution and is the most stable structure of polyoxometalate catalysts.

In a preferred embodiment, the invention provides a process according to the first aspect of the invention, whereby said heteropoly acid is supported on a support material, whereby said support material is an inorganic support material. Examples of inorganic support materials are, but not limited to, silica, zeolites such as but not limited to natural zeolites such as chabazite, erionite, mordenite and faujasite and synthetic zeolites, alumina, clay, zirconia, ceria, carbon, zinc oxides, titanium oxides, and/or salts thereof. The use of a support material is of interest because of their thermal and mechanical stability and resistance, their availability through their ease of synthesis, the ease of synthesis of catalyst on support, their large surface area, resistance to corrosion and good bonding properties with heteropoly acid compounds.

Nevertheless, when supporting heteropoly acids, a homogeneous dispersion of the active phase improves catalytic activity. Hereby, a good distribution of catalyst on the support phase was obtained, whereby in a preferred embodiment the catalyst was predominantly present as a monolayer, fully covering the underlying support. On the other hand, a higher loading resulted in a bulk-like behaviour, whereas a lower loading led to non-homogeneous dispersion. Preferably, said catalyst comprises one or more strongly acidic and weakly acidic sites. This is advantageous since the proximity of strongly and weakly acidic sites promotes the formation of intermediate organic structures, thereby lowering the activation energy for the formation of the desired end-product, resulting in enhanced conversion, selectivity and yield. Use of inorganic catalysts is advantageous since they can be easily recycled after removing the formed cokes by heating and oxidation of said cokes.

In a preferred embodiment, the invention provides a process according to the first aspect of the invention, whereby said heteropoly acid is supported on a support material, whereby said support material preferably comprises silica and/or alumina, preferably silica, and more preferably mesoporous silica.

In a preferred embodiment, the invention provides a process according to the first aspect of the invention, whereby said heteropoly acid is used in a fixed bed reactor, in the form of pellets, tablets, granulates, extrudates, monoliths, or similar structures.

In a preferred embodiment, said process is performed in a reactor such as known to those skilled in the art including, but not limited to, a batch reactor, a stirred tank reactor, a semi-batch reactor, a continuous reactor, a continuous stirred tank reactor, a slurry reactor, a fixed bed reactor, a tubular reactor, a column reactor, a packed bed reactor, a fluidized bed reactor, a trickle bed reactor, a membrane reactor, a plate and frame reactor, a Carberry-type reactor, a plug flow reactor, and a reactive distillation, or various combinations of any thereof. It will be understood that the manner in which the alcohol and the aminating agent are fed/added into the reactor can vary depending on the equipment used and the phase of each reaction component.

The heteropoly acid can be used as a bulk compound or as supported heteropoly acid. The invention can be advantageously performed using a support material with a surface area between 100 and 2500 m²/g, more preferably between 150 and 1000 m²/g. The reactivity of heterogeneous catalysts occurs at the surface atoms. The support may be inert or may participate in the catalytic reactions. The supports may include various kinds of carbon, alumina, titanium oxide and/or silica. Use of a supported heteropoly acids has as effect that the product is easily separated from the catalyst. This has the advantage that the process is economically more relevant.

The textural properties of the catalysts were determined from N₂ adsorption-desorption isotherms recorded at the liquid nitrogen temperature using an ASAP 2010 Micromeritics apparatus after outgassing the solids at 130°C for 2 h. The specific surface area, the pore volume, and the pore size distribution were obtained by BET and BJH methods, respectively.

In a preferred embodiment, the invention provides a process according to the first aspect of the invention, whereby said heteropoly acid is comprised in an amount of 5-90% by weight relative to the total weight of the supported catalyst, preferably 30-70% by weight.

In a preferred embodiment, the invention provides a process according to the first aspect of the invention, wherein said heteropoly acid is selected from the group of Cs₃PMo₁₂O₄₀, CS₃PW₁₂O₄₀, Cs₄PMo₁₁VO₄₀, CS₂HPMo₁₂O₄₀ and/or CS_{2.5}H_{0.5}PMo₁₂O₄₀.

The acid properties of the catalysts were determined by thermo-programmed desorption of NH₃ (NH₃-TPD). A typical NH₃-TPD test was carried out as follows: 50 mg of catalyst were pre-treated in a He flow (30 mL/min) at 250°C for 2 h in order to remove the physisorbed and crystal water. Then, the sample was cooled to 130°C, and NH₃ pulsed injections were carried out until saturation. The TPD profiles were monitored using a TCD (Thermal Conductivity Detector) and a mass-spectrometer as detectors, and recorded from 130 to 700°C at a 10°C/min heating rate.

Raman spectroscopy is to be understood as a spectroscopic technique used to observe vibrational, rotational, and other low-frequency modes in a system. Raman spectra were recorded on a Jobin-Yvon LabRam Infinity apparatus equipped with a CCD (Charge Coupled Device) detector operating at the liquid nitrogen temperature. A D2 filter was used to protect the catalyst structure from thermal degradation by the laser beam heat (λ = 532 nm). The spectra were recorded in the range between 200 and 1400 cm⁻¹. The homogeneity of the samples was checked by performing the analysis on at least three different particles for each sample.

Elemental analysis is a method where a sample of some material is analysed for its elemental and sometimes isotopic composition. Elemental analysis was performed by energy dispersive X-ray spectroscopy (EDS) on a Hitachi S3600N electron microscope equipped with a Thermo Ultradry EDS detector using an acceleration voltage of 25 kV.

The use of polyols has driven a specific attention, due to the demand in diamines and polyamines, especially ethylene diamine and its polymers. It is desired to obtain sufficiently high selectivity to the desired product. Already with monofunctional alcohols side reactions can occur. Difficulties are amplified when using a di- or polyfunctional alcohols or aminoalcohols as reactant and in many cases the selectivity to the desired product is far below the economically acceptable level.

In a preferred embodiment, the invention provides a process according to the first aspect of the invention, wherein said alcohol is a polyol comprising a 1,2-diol moiety. In a more preferred embodiment, the invention provides a process according to the first aspect of the invention, wherein said polyol is glycerol.

In a preferred embodiment, glycerol is provided as crude glycerol comprising water and glycerol, more preferably comprising water, glycerol and salts. Most preferably glycerol is provided as crude glycerol comprising glycerol, between 5 wt.-% and 25 wt.-% of water and between 1 wt.-% and 10 wt.-% of salts. In a preferred embodiment, said crude glycerol is fed to said process after partial or complete separation from water and salts using industrial evaporators. This is advantageous since crude glycerol is widely available in the industry and can thus be advantageously converted without expensive purification methods.

Use of glycerol as raw material for direct amination is of interest because it offers a valorisation for glycerol, being available in large quantities from renewable resources these days. Finding new uses for glycerol has become a key issue in order to balance the overall economic viability of the biodiesel industry.

At least one embodiment of the present invention provides access to glycerol-based specialty amines. These specialty amines are amines which are characterized in that at least one hydroxyl group of the glycerol has been substituted for a primary amino group, a secondary amino group or a tertiary amino group, for example 1,2,3-triaminopropane, 1,3-diaminopropan-2-ol, 1,2-diaminopropan-3-ol, 3-aminopropane-1,2-diol or 2-aminopropane-1,3-diol. These compounds have a high number of functionalities and may therefore be important intermediates in the synthesis of organic compounds, such as crop protection compositions, pharmaceuticals and stabilizers.

Other important synthetic intermediates obtainable from glycerol are ketones or aldehydes. An example in the reaction of glycerol with dimethyl amine is for instance N,N-dimethylaminoacetone, which can be easily converted by known procedures to 1-(N,N-dimethylamino)-propan-2-ol through hydrogenation or to 1-(N,N-dimethylamino)-2-aminopropane through reductive amination.

In a preferred embodiment, the invention provides a process according to the first aspect of the invention, wherein said aminating agent is ammonia or an alkylamine.

In a preferred embodiment, the invention provides a process according to the first aspect of the invention, wherein said alkylamine comprises one or more monoalkylamines with a general structure of H₂NR1, whereby R1 is a C1-C6 alkyl, preferably CH₃; and/or one or more dialkylamines with a general structure of HNR1R2, whereby R1 is a C1-C6 alkyl, preferably CH₃ and whereby R2 is a C1-C6 alkyl, preferably CH₃.

For example, the following mono- and dialkylamines may be used as aminating agents: methylamine, dimethylamine, ethylamine, diethylamine, n-propylamine, di-n-propylamine, isopropylamine, diisopropylamine, isopropylethylamine, n-butylamine, di-n-butylamine, s-butylamine, di-s-butylamine, isobutylamine, n-pentylamine, s-pentyl-amine, isopentylamine, n-hexylamine, s-hexylamine, isohexylamine, and cyclohexylamine.

In a more preferred embodiment, said dialkylamine is dimethylamine. This is of interest because dimethylamine is inexpensive and available on the industrial scale.

In a preferred embodiment, the invention provides a process according to the first aspect of the invention, wherein the molar ratio of said aminating agent to said alcohol is between 10:1 and 1:1. In a more preferred embodiment, said molar ratio of the amine compound to alcohol is from about 4:1 to about 2:1.

In a preferred embodiment, the invention provides a process according to the first aspect of the invention, wherein said amination comprises a reaction temperature from 100 to 400°C.

In a more preferred embodiment, said temperature is between 200°C and 300°C, more preferably between 230°C and 280°C. Most preferably, said temperature is 230°C, 240°C, 250°C, 260°C, 270°C or 280°C, or any temperature there in between. Interestingly, the inventors have found that heteropoly acids can be successfully employed in the amination process according to the invention, showing high catalytic activity even at these relatively low temperatures. This is advantageous because performing the reaction at a lower temperature requires less energetic input.

In view of the above, it will be understood that reaction conditions can vary depending on the particular reaction components (i.e. alcohol, aminating agent, heteropoly acid and gas, if present) and reactor type selected. In certain embodiments, reacting the alcohol with the heteropoly acid catalyst may occur at a temperature of from about 160°C to about 300°C, and in another embodiment from about 200°C to about 240°C. According to certain embodiments, reacting the alcohol with the heteropoly acid may occur at about atmospheric pressure, although pressures above and below atmospheric pressure, for example in one embodiment, pressures from about 0.1 bar to about 60 bar may be used herein and in another embodiment, pressures from about 0.1 bar to about 10 bar, may be used herein. Similarly, the time needed to carry out the reaction can vary depending on the reaction components used, i.e. the reaction time may be from about 1 second to about 1 hour. Those skilled in the art will understand how to select the proper process parameters based on such factors as the reaction components, reactant phase, and equipment used.

After the reaction, the desired reaction products can be separated from the reaction mixture in the reactor, that is, the aminated product may be separated so as to obtain the individual products (i.e. unreacted adduct, unreacted amine, impurities, water, ...). For example, streams of unreacted adduct, and unreacted amine may be recycled for reuse to save on raw material costs. Water and impurities may be considered by-products of the reaction and, thus, can be separated and removed from the other reaction products and either processed for further use in another application (propylene glycol), recycled, or disposed (water and impurities). The separation process may include any separation process known in the art, such as, but not limited to, flash distillation, fractional distillation, chromatography, extraction, passing through an acidic resin, and combinations of any thereof.

Finally, the aminated product may be collected as the desired product for use in a variety of applications as, for example as solvents, intermediates for making surface active agents, corrosion inhibitors in metal working fluids, neutralizing agents in acid scrubbing during natural gas or syngas purification processes, and aids in the preparation of compounds in the pharmaceutical industry. As will be understood by those skilled in the art, the specific separation processes used and the degree of separation may depend on the desired purity of the reaction products.

In a preferred embodiment, the invention provides a process according to the first aspect of the invention, wherein said alcohol is glycerol and wherein said aminating agent is an alkylamine or dialkylamine, thereby obtaining alkylamino (1-alkylamino propanone) or dialkylamino acetone (1-dialkylamino-2-propanone), and wherein said alkylamino or dialkylamino acetone is further reductively aminated.

In a more preferred embodiment, the invention provides a process according to the first aspect of the invention, wherein said alcohol is glycerol and wherein said aminating agent is dimethylamine, thereby obtaining dimethylamino acetone, and wherein said dimethylamino acetone is further reductively aminated. One of the advantages of said process is to provide an economically viable route to 1-dimethylamino-2-aminopropane. Preferably, dimethylamino acetone, obtained by a process according to the first aspect of the invention is contacted with an aminating agent, preferably ammonium NH₃, under an atmosphere of hydrogen gas H₂ in presence of a reduction catalyst.

Preferably, the ratio of aminating agent to ketone is comprised between 1 and 32, more preferably between 4 and 16 and most preferably, said ratio is equal to 6, 7, 8, 9, 10, 11, 12, 13 or 14 or any ratio there in between. Preferably, said process is performed at a temperature between 50°C and 120°C, more preferably between 60°C and 100°C, and even more preferably between 70°C and 90°C. Most preferably, said process is performed at a temperature of 70°C, 75°C, 80°C, 85°C or 90°C. Preferably, said process is performed at a hydrogen H₂ pressure of between 5 bar and 100 bar, more preferably between 20 bar and 60 bar and even more preferably between 30 bar and 50 bar. Most preferably, said process is performed at a hydrogen H₂ pressure of 30 bar, 35 bar, 40 bar, 45 bar or 50 bar, or any pressure there in between. Preferably, said process is performed during a reaction time of between 0.5 h and 24 h, more preferably between 1 h and 4 h and even more preferably between 1 h and 3 h. Said reduction catalyst can be any reduction catalyst known in the state of the art such as, but not limited to, Pd/C, Raney Nickel and Raney Copper.

In a second aspect, the present invention provides a use of an amine obtained by a process according to the first aspect of the invention as a synthetic unit for the production of surfactants, pharmaceuticals, crop protection compositions, stabilizers, polymers, hardeners for epoxy resins, catalysts for polyurethanes, intermediates for preparing quaternary ammonium compounds, plasticizers, corrosion inhibitors, synthetic resins, ion exchangers, textile assistants, dyes, vulcanization accelerators and/or emulsifiers.

In a further aspect, the present invention also pertains to the products obtained by a process according to the first aspect of the invention and to any uses thereof.

### EXAMPLES

In the following examples are intended to further clarify the present invention, and are nowhere intended to limit the scope of the present invention. A process according to the invention is illustrated by the examples which follow.

### EXAMPLE 1

A heteropoly acid Cs₃PMo₁₂O₄₀, was prepared by a precipitation method. An aqueous solution of Cs₂CO₃ (0.1 M, Fluka) was added *via* a liquid pump into an aqueous solution of H₃PMo₁₂O₄₀ (0.1 M). The temperature of the mixture was maintained at 45°C under vigorous stirring. After 2 h, the solid was recovered by removing the solvent under reduced pressure. The sample was then dried at 70°C for 24 h.

A direct amination of glycerol was carried out in a fixed bed down-flow reactor of 110 mm height and 8 mm internal diameter, with a catalyst bed with 1 g of catalyst, set at the middle of the reactor, in the isothermal zone. The temperature was controlled by a thermocouple located near the catalyst bed. Nitrogen was used as a carrier gas at a flow rate of 30 mL.min⁻¹, which was controlled by a mass flow controller (Brooks). Glycerol and dimethylamine (DMA) aqueous solutions (40 %wt. each) were fed *via* two HPLC pumps (GILSON) with a flow rate of 0.1 mL.min⁻¹ and 2.4 mL.min⁻¹, respectively, and evaporated upfront the reactor at 230°C. The molar ratio of glycerol: DMA: N₂: H₂O was 6.1%:6.4%:17.2%:70.4%. The reaction temperature was fixed to 290°C. The reaction products together with unconverted glycerol were collected each two hours in a trap at room temperature and analysed offline using gas chromatography and high-performance liquid chromatography. GC analyses were performed on an Agilent 6890 GC using a flame ionization detector and equipped with a Zebron CP Sil 5 CB semi-capillary column (30 m length, film thickness 3 µm, internal diameter of 0.53 mm). High Performance Liquid Chromatography (HPLC) was used to quantify the amount of glycerol using a Refractory index detector (Shodex RI-101) and a Rezex ROA-organic column (Phenomenex, 300 x 7.80 mm).

Use of the Cs₃PMo₁₂O₄₀ catalyst for the direct amination of glycerol using DMA showed high selectivity to dimethylamino-2-propanone (DMA acetone) (29 %), but low conversion (25 %), as can be seen from the overview presented in Table 1.

### EXAMPLE 2

Conditions for this example were analogous to Example 1, except for the fact that Cs₃PW₁₂O₄₀ was selected as heteropoly acid. The precursor of Cs₃PW₁₂O₄₀, phosphotungstic acid (H₃PW₁₂O₄₀) is commercially available as crystalline hydrate.

The direct amination of glycerol using DMA was executed as described in Example 1. The direct amination using Cs₃PW₁₂O₄₀ as catalyst showed selectivity to two main products: DMA acetone and 3-dimethylamino-1,2-propanediol with 11% and 7%, respectively, with a conversion of 20%, as presented in Table 1.

### EXAMPLE 3

Conditions for this example were analogous to Example 1, except for the fact that Cs₄PMo₁₁VO₄₀ was selected as heteropoly acid for the direct amination of glycerol with DMA.

The precursor of Cs₄PMo₁₁VO₄₀, vanado-11-molybdic acid (H₄PMo₁₁VO₄₀), was prepared as follows: 7.1 g of Na₂HPO₄ were dissolved in 100 mL of water. Then, a solution comprising 6.1 g sodium metavanadate dissolved in 100 mL of boiling water was added. The mixture was then left to cool to room temperature before being acidified with 5 mL of concentrated sulfuric acid. Then, a solution containing 133 g of Na₂MoO₄.2H₂O dissolved in 200 mL water was added, before further slow addition of 85 mL of concentrated sulfuric acid under vigorous stirring. A light red solution was obtained. The heteropoly acid was then extracted with 400 mL of diethyl ether. After evaporation of the organic solvent under vacuum at 60°C, the obtained orange solid was recrystallized in water.

The direct amination of glycerol using DMA was executed as described in Example 1. The catalytic results for Cs₄PMo₁₁VO₄₀ showed 4% selectivity to DMA acetone, with a conversion of 43%, as summarized in Table 1.

### EXAMPLE 4

Conditions for this example were analogous to Example 1, except for the fact that Cs₂HPMo₁₂O₄₀ was selected as heteropoly acid for the direct amination of glycerol with DMA. The precursor for Cs₂HPMo₁₂O₄₀, phosphomolybdic acid (H₃PMo₁₂O₄₀), is commercially available as crystalline hydrate.

The direct amination of glycerol with DMA was executed as described in Example 1. The catalytic results for Cs₂HPMo₁₂O₄₀ exhibited 28% selectivity to DMA acetone with a conversion of 11%, as summarized in Table 1.

### EXAMPLE 5

Conditions for this example were analogous to Example 1, except for the fact that Cs_{2.5}H_{0.5}PMo₁₂O₄₀ was used as heteropoly acid for the direct amination of glycerol with DMA. The precursor for Cs₂HPMo₁₂O₄₀, phosphomolybdic acid (H₃PMo₁₂O₄₀), is commercially available as crystalline hydrate.

The direct amination of glycerol with DMA was executed as described in Example 1. The catalytic results for Cs_{2.5}H_{0.5}PMo₁₂O₄₀, gave a selectivity of the two main products, DMA acetone and 3-DMA-1,2-propanediol, of 63% with a glycerol conversion of 8%. The results are listed in Table 1.

**Table 1: Direct amination of glycerol by a heteropoly acid catalyst under the following conditions: T = 290°C, P = 1 bar.**

| Example | Catalyst | Conversion (%) | Selectivity (DMA acetone : 3-DMA-1,2-propandiol) |
|---|---|---|---|
| 1 | Cs₃PMo₁₂O₄₀ | 25 | > 95 |
| 2 | Cs₃PW₁₂O₄₀ | 20 | 50 - 70 |
| 3 | Cs₄PMo₁₁VO₄₀ | 43 | > 95 |
| 4 | Cs₂HPMo₁₂O₄₀ | 11 | > 95 |
| 5 | Cs_{2.5}H_{0.5}PMo₁₂O₄₀ | 8 | > 95 |

### EXAMPLE 6-8 AND COMPARATIVE EXAMPLE 1 AND 2

In additional examples 6-8, a process for the direct amination is performed under conditions analogous to Example 1, except for the fact that the following catalysts for the direct amination of glycerol with dimethylamine were used: H₃SiW₁₂O₄₀ (example 6), H₄SiMo₁₂O₄₀ (example 7), NdHPO₄ (comparative example 1), LaHPO₄ (comparative example 2) and using Cs₄PMo₁₁VO₄₀ for the direct amination of crude glycerol (example 8). The catalysts of Comparative example 1 and 2 are prepared according to literature reports, US 4,578,517 and do not qualify as heteropoly acids as defined in the present invention. The amination is carried out as described under example 1. The results are summarized in Table 2.

**Table 2: Direct amination of glycerol by a heteropoly acid catalyst under the following conditions: T = 290°C, P = 1 bar.**

| Example | Catalyst | Conversion (%) | Selectivity (DMA acetone : 3-DMA-1,2-propandiol) |
|---|---|---|---|
| 6 | H₃SiW₁₂O₄₀ | +++ | +++ |
| 7 | H₄SiMO₁₂O₄₀ | +++ | +++ |
| 8^{(*)} | Cs₄PMo₁₁VO₄₀ | ++ | +++ |
| Comparative example | Catalyst | Conversion (%) | Selectivity (DMA acetone : 3-DMA-1,2-propandiol) |
| 1 | NdHPO₄ | + | - |
| 2 | LaHPO₄ | - | + |

| | | | |
|---|---|---|---|
| +++ very good (conversion > 45%; selectivity > 65% to DMA acetone) ++ good (conversion 30-45%; selectivity 25-65%) + sufficient (rather low conversion of 10-30%; selectivity 5-25%) - insufficient (low conversion < 10%; selectivity < 5% to DMA acetone) (*) glycerol was employed as crude glycerol comprising 20 wt.-% water and 5 wt.-% salts and was fed to the reactor after treatment in an industrial evaporator | | | |

### EXAMPLE 9-11

The preparation of Cs_{2.5}H_{0.5}PMo₁₂O₄₀ supported on silica was carried out in two steps: First, the silica carrier was impregnated with cesium carbonate: 2.5 g of silica support (CARIACT Q-10, from Fuji Silysia; 272 m²/g; 1.26 cm³/g pore volume; average pore diameter of 14.9 nm) and 30 mL of ethanol were stirred at 60°C. Then, the desired amount of cesium carbonate (0.1 M, Fluka) was added. After 30 minutes, the solvent was evaporated, and the solid was dried at 60°C for 2 h. The as-prepared silica was then impregnated with commercial phosphomolybdic acid. The support was suspended in 30 mL of extra-dry ethanol. The required amount of HPA dissolved in a minimum quantity of ethanol was subsequently added. After 30 min, the solvent was evaporated under vacuum at 60°C, and the obtained solid was dried at 50°C for two hours. Catalysts supported on silica with nominal loadings of 30, 50 and 70 %wt. of Cs_{2.5}H_{0.5}PMo₁₂O₄₀ were prepared, and the corresponding samples were labelled 30CsPMo, 50CsPMo and 70CsPMo, respectively.

The direct amination of glycerol with DMA was executed as described in Example 1. The catalytic results for silica supported Cs_{2.5}H_{0.5}PMo₁₂O₄₀, gave a selectivity of the two main products, DMA acetone and 3-DMA-1,2-propandiol, of 2-33% with a glycerol conversion 8-61%, according to the amount of Cs_{2.5}H_{0.5}PMo₁₂O₄₀ in the catalyst. The results are listed in Table 3.

**Table 3: Direct amination of glycerol by a supported heteropoly acid catalyst under the following conditions: T = 290°C, P = 1 bar.**

| Example | Catalyst | Conversion (%) | Selectivity (DMA acetone : 3-DMA-1,2-propandiol) |
|---|---|---|---|
| 9 | Cs_{2.5}H_{0.5}PMo₁₂O₄₀ / silica (30%w) | 61 | > 90 |
| 10 | Cs_{2.5}H_{0.5}PMo₁₂O₄₀ / silica (50%w) | 33 | > 90 |
| 11 | Cs_{2.5}H_{0.5}PMo₁₂O₄₀ / silica (70%w) | 8 | > 90 |

### EXAMPLE 12-14

Further examples illustrate the further use of the obtained dimethylamino acetone in a subsequent reductive amination process. Dimethylamino acetone used in the experiments of Example 12-14 was not distilled prior to use, and was obtained according to the procedure of Example 3. Reaction conditions and the conversion of dimethylamino acetone to 1-dimethylamino-2-aminopropane are depicted in Table 4. The results show that high conversions was obtained for all tested reaction conditions. Finally, 1-dimethylamino-2-aminopropane was isolated via distillation in high yields.

**Table 4: Reductive amination of dimethylamino acetone (DMA) in presence of Raney Nickel catalyst.**

| Example | Ratio NH₃/ketone | Temperature (°C) | Pressure H₂ (bar) | Time (h) | Conversion (%) |
|---|---|---|---|---|---|
| 12 | 11.5 | 90 | 40 | 2 | 95 |
| 13 | 5.3 | 80 | 43 | 2.25 | 93 |
| 14 | 8.0 | 75 | 45 | 3 | 93 |

## Claims

1. Process for preparing amines by reacting an alcohol with an aminating agent in presence of a catalyst, **characterized, in that** said catalyst is a heteropoly acid comprising one or more central atoms (X) and addenda atoms (M, N), whereby said addenda atoms comprise one or more transition metals, and/or a salt of said heteropoly acid.

2. Process according to claim 1, wherein said addenda atoms are selected from group 5, group 6 or group 7 of the periodic table.

3. Process according to claim 2, wherein said addenda atoms are selected from the group consisting of tungsten, molybdenum and vanadium, or any mixture thereof.

4. Process according to any of the previous claims, wherein said central atoms (X) comprise one or more central atoms selected from group 14 or group 15 of the periodic table, or any mixture thereof.

5. Process according to claim 5, wherein said central atoms (X) are selected from the group consisting of silicon, phosphorus, germanium and arsenic, or any mixture thereof.

6. Process according to any of the previous claims, wherein said salt of said heteropoly acid comprises an element selected from group 1 and/or group 2, preferably rubidium and/or cesium.

7. Process according to any of the previous claims, whereby said heteropoly acid is provided as a Keggin structure.

8. Process according to any of the previous claims, whereby said heteropoly acid is supported on a support material, whereby said support material preferably comprises silica and/or alumina.

9. Process according to claim 8, whereby said heteropoly acid is comprised in an amount of 5-90% by weight relative to the total weight of the supported catalyst, preferably 30-70% by weight.

10. Process according to any of the previous claims, wherein said heteropoly acid is selected from the group of Cs₃PMo₁₂O₄₀, Cs₃PW₁₂O₄₀, Cs₄PMo₁₁VO₄₀, Cs₂HPMo₁₂O₄₀ and/or Cs_{2.5}H_{0.5}PMo₁₂O₄₀.

11. Process according to any of the previous claims, wherein said alcohol is a polyol.

12. Process according to claim 11, wherein said polyol is selected from the group of glycerol, ethylene glycol, propylene glycol, diethylene glycol and ethanolamine.

13. Process according to any of the previous claims, wherein said aminating agent is ammonia or an alkylamine.

14. Process according to any of the previous claims, wherein said alkylamine comprises one or more monoalkylamines with a general structure of H₂NR1, whereby R1 is a C1-C6 alkyl, preferably CH₃; and/or one or more dialkylamines with a general structure of HNR1R2, whereby R1 is a C1-C6 alkyl, preferably CH₃ and whereby R2 is a C1-C6 alkyl, preferably CH₃.

15. Process according to any of the previous claims, wherein said alcohol is glycerol and wherein said aminating agent is dimethylamine, thereby obtaining dimethylamino acetone, and wherein said dimethylamino acetone is further reductively aminated.
